# EUROPEAN PATENT APPLICATION

(11) **EP 4 141 013 A1**
(43) Date of publication of application: **01.03.2023**
(21) Application number: 21194182.8
(22) Date of filing: 31.08.2021
(51) Int. Cl.: C07F 7/18

(54) **STEREOCHEMICALLY PURE LIPIDS FOR NUCLEIC ACID DELIVERY**

(71) Applicant: Studiengesellschaft Kohle mbH, 45470 Mülheim (DE)
(72) Inventor: LIST, Benjamin, 45470 Mülheim an der Ruhr (DE); DE, Chandra Kanta, 45468 Mülheim an der Ruhr (DE); ZHU, Chendan, 45468 Mülheim an der Ruhr (DE); DEHN, Stefanie, 45326 Essen (DE); HINRICHS, Heike, 45470 Mülheim an der Ruhr (DE)

(57) **Abstract**

The present application generally relates to stereochemically pure lipids that can be used, also in combination with other lipid components, such as neutral lipids, cholesterol and polymer conjugated lipids, to form lipid nanoparticles with oligonucleotides, to facilitate the intracellular delivery of therapeutic nucleic acids such as oligonucleotides, messenger RNA both in vitro and in vivo.

## Description

The present application generally relates to stereochemically pure lipids that can be used, also in combination with other lipid components, such as neutral lipids, cholesterol and polymer conjugated lipids, to form lipid nanoparticles with oligonucleotides, to facilitate the intracellular delivery of therapeutic nucleic acids such as oligonucleotides, messenger RNA both in vitro and in vivo.

In more detail, embodiments of the present invention generally relate to the preparation and characterization of stereochemically pure lipids to be used in lipid nanoparticles that facilitate or enable the delivery of pharmaceutically active compounds such as nucleic acids (e.g., oligonucleotides, messenger RNA) into cells.

There is a significant potential of nucleic acids (e.g., oligonucleotides, messenger RNA) as therapeutic agents. However, two problems currently face the use of oligonucleotides in therapeutic contexts. First, free RNAs are susceptible to nuclease digestion in plasma. Second, free RNAs have limited ability to gain access to the intracellular compartment where the relevant translation machinery resides. To address this issue, lipid nanoparticles have been designed and developed that facilitate intracellular delivery of the nucleic acid pharmaceutical. One constituent of these lipid nanoparticles (LNP) are synthetic lipids that contain amines, which under physiological conditions, are protonated and therefore positively charged. Several lipids of that kind are disclosed in WO2017075531 or WO2018081480 of Acuitas Therapeutics Inc., exemplified by Acuitas-5.

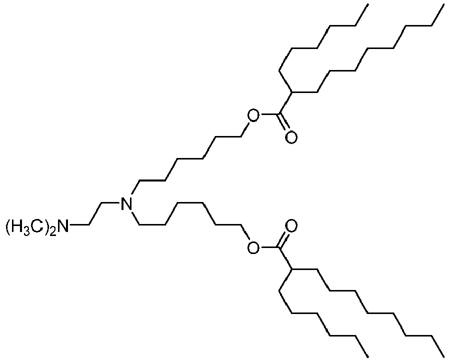

Another specific frequently used example is ALC - 0315, which has found prominent utility in mRNA vaccine delivery, among other applications.

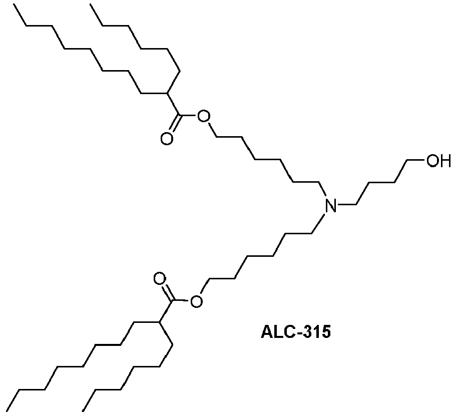

Remarkably, one important aspect of this lipid class has never been paid attention to: they exist in multiple stereoisomeric forms. Consequently, the synthetic lipids that have previously been incorporated into LNPs were prepared and used as mixtures of several stereoisomeric forms. In the state of the art, no synthesis or descriptions of stereochemically pure synthetic lipids to be used in NLPs have been disclosed. Pathways to such enantiopure lipids have previously not been disclosed.

ALC-0315, as a typical example, contains two stereogenic centers, which are sp3-hybridized carbon atoms that are surrounded by four different substituents. As a consequence, ALC-0315 exists in three stereoisomeric forms, two chiral enantiomers (R,R)-ALC-0315 and (S,S)-ALC-0315, and one so-called meso-compound (R,S)-ALC-0315, which is achiral. None of these isomers has previously been mentioned, characterized or preparateively accessed, either synthetically or chromatographically, or by any other means.

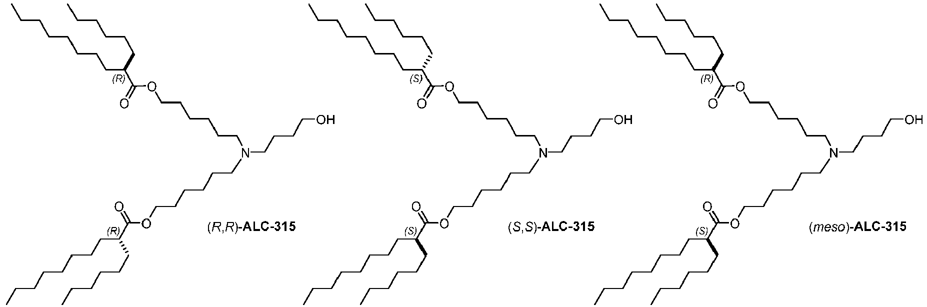

It is well known that the human body is very capable of differentiating stereoisomers. As a consequence, the vast majority of all modern pharmaceuticals are normally used as single isomer.

The inventors therefore suggest here that the ALC-0315 stereoisomers as well as related lipids will similarly differ with regard to their ability (a) to prevent nucleic acid degradation and (b) to deliver the nucleic acids locally or systemically and intracellularily, Furthermore, (c) the inventors anticipate the stereoisomers to significantly differ with regard to tolerance, therapeutic index, and toxicity.

Thus, there was a need in the state of the art to provide a process, for example through synthesis or resolution, to enantiopure lipids .

This patent application therefore is directed to the separation, preparation and characterization of stereochemically pure forms of NLPs such as ALC-315.

In more detail, the present invention is directed to a lipid compound as represented by the following Formula (I): 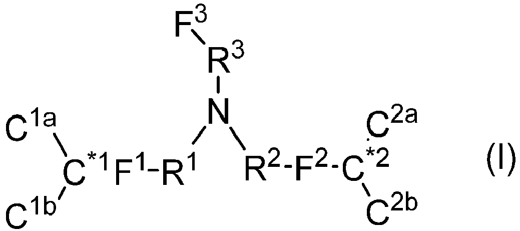 or a pharmaceutically acceptable salt thereof, wherein:
C*¹ and C*² are each representing a CH group;
C^{1a} and C^{1b} are different from each other and independently represent C₆-C₂₄ alkyl or C₆-C24 alkenyl;
C^{2a} and C^{2b} are different from each other and independently represent C₆-C₂₄ alkyl or C₆-C₂₄ alkenyl;
C^{1a} is the same or different as C^{2a} and C^{1b} is the same or different as C^{2b};
R¹ and R² are each unsubstituted C₁-C₁₂ alkylene or C₂-C₁₂ alkenylene, R¹ and R² preferably representing the same group;
R³ is C₁-C₂₄ alkylene, C₂-C₂₄ alkenylene, C₃-C₈ cycloalkylene, or C₃-C₈ cycloalkenylene;
F¹ and F² are each selected from -O(C=O)-, -(C=O)O-, -C(=O)-, -O-, -S(O)_{1,2}-, -S-S-, - C(=O)S-, SC(=O)-, - RC(=O)-, -C(=O)R-, RC(=O)R-, -OC(=O)R-, - RC(=O)O- or a direct bond, wherein R being H or C₁ to C₁₂ alkyl, F¹ and F² preferably representing the same group and more preferably being -O(C=O)- or -(C=O)O-, respectively;
F³ is H, OR⁴, -NR⁴₂, -CN, halogen, -C(=O)O-(C₁-C₁₂ alkyl)-, (C₁-C₁₂ alkyl)-OC(=O)- (C₁-C₁₂ alkyl)- or - R⁴C(=0)-(C₁-C₁₂ alkyl)-, R⁴ being H or C₁ to C₆ alkyl,
wherein the stereogenic centers at C*¹ and C*² are independently from each other enriched in one stereogenic form selected from the (R)-form or the (S)-form.

According to the present invention, it is possible to prepare a lipid compound wherein the stereogenic centers are independently from each other enriched in one stereogenic form selected from the (R)-form or the (S)-form. Enriched in the sense of the invention means that a stereogenic center being generally present in the (R)-form or the (S)-form is predominantly in one of those forms, starting at a ratio of more than 50:50, preferably more than 60:40, more preferably more than 70/30, even more preferred 80:20 or even more than 90:10 up and ideally in a ratio of 100:0 of one of the two forms.

It should be clear to the skilled man that, depending on the kind of the hydrocarbon substituents in Formula (I), more than two stereogenic centers may be present in one compound of Formula (I) so that a complex mixture may be obtained, particularly if prepared according to the state of art. Therefore, it is highly desirable to use enantiopure starting materials to obtain an enantiopure product and to reduce the percentage of the racemic mixture.

In one embodiment of the invention, in Formula (I), C^{1a} and C^{2a} represent the same group selected from C₆-C₂₄ alkyl or C₆-C₂₄ alkenyl, and C^{1b} and C^{2b} represent the same group selected from C₆-C₂₄ alkyl or C₆-C₂₄ alkenyl in Formula (I). Thus, the number can be reduced in order to facilitate the synthesis of an enantiopure product.

Any functional group selected from -O(C=O)-, -(C=O)O-, -C(=O)-, -O-, -S(O)_{1,2}-, -S-S-, - C(=O)S-, SC(=O)-, - RC(=O)-, -C(=O)R-, RC(=O)R-, -OC(=O)R- or - RC(=O)O-, or a direct bond, wherein R being H or C₁ to C₁₂ alkyl, preferably -O(C=O)- or -(C=O)O-, respectively, may be present in the inventive lipids.

In a further embodiment of the lipid compound, R¹ and R² are the same and each unsubstituted C₁-C₁₂ alkylene in Formula (I).

In another embodiment of the the lipid compound, R³ is C₁-C₂₄ alkylene.

The lipid compound as claimed in any one of the preceding claims, wherein, in Formula (I), F³ is OR⁴, -NR⁴₂, -CN, -or halogen, R⁴ being H or C₁ to C₆ alkyl.

A particular lipid compound is represented by Formula (I), wherein
C*¹ and C^{*2} are each representing a CH group;
C^{1a} and C^{2a} represent the same C₆-C₂₄ alkyl group;
C^{1b} and C^{2b} represent the same C₆-C₂₄ alkyl group;
F¹ and F² each represent the same group selected from -O(C=O)-, -(C=O)O-;
R¹ and R² are each C₄₋₈ alkylene;
R³ is C₁-C₂₄ alkylene; and
F³ is OR⁴, or -NR⁴₂, R⁴ being H or C₁ to C₆ alkyl,
wherein the stereogenic centers at C*¹ and C*² are independently from each other enriched in one stereogenic form selected from the (R)-form or the (S)-form. For said lipid compound, the stereogenic centers are more particularly in the (R)-form as (R,R) or in the (S)-form as (S,S), and the enantiopure forms are preferred.

The lipid compound, wherein, in Formula (I),
C*¹ and C^{*2} are each representing a CH group;
C^{1a} and C^{2a} each represent a C₆ alkyl group;
C^{1b} and C^{2b} each represent a C₈ alkyl group;
F1 and F2 each represent -O(C=O)- group bound to C*^{(1,2)};
R¹ and R² are each unsubstituted C₄₋₈ alkylene, preferably C₆ alkylene;
R³ is C₂ to C₄ alkylene; and
F³ is OR⁴, or -NR⁴₂, R⁴ being H or a methyl group,
wherein the stereogenic centers at C*¹ and C*² are independently from each other enriched in one stereogenic form selected from the (R)-form or the (S)-form, is of particular importance in view of its identical hydrocarbon groups, thus, depending on the optical purity of the starting compounds, representing the lipid compound in the enantiopure (R,R) or (S,S) forms or the meso form (R,S) .

The present invention is also directed to a process for preparing a lipid compound as represented by the Formula (I): 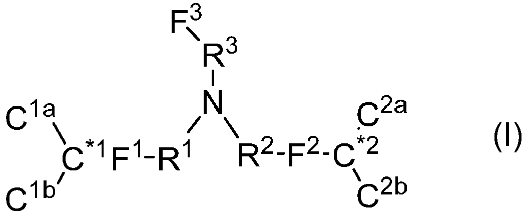 wherein, in Formula (I),
C*¹ and C^{*2} are each representing a CH group;
C^{1a} and C^{2a} represent the same group selected from C₆-C₂₄ alkyl, and C^{1b} and C^{2b} represent the same group selected from C₆-C₂₄ alkyl;
F¹ and F² each represent the same group selected from -O(C=O)-, -(C=O)O-;
R¹ and R² are each C₄₋₈ alkylene;
R³ is C₁-C₂₄ alkylene; and
F³ is OR⁴, or -NR⁴₂, R⁴ being H or C₁ to C₆ alkyl,
wherein the stereogenic centers at C*¹ and C*² are independently from each other enriched in one stereogenic form selected from the (R)-form or the (S)-form,
wherein a X-C₁-C₂₄ alkylene-NH₂, wherein X is an -OY or -NR⁴₂, Y being a protective group and being preferably selected from trialkylsilyl, MOM (methoxymethyl ether), BOC and others, and R⁴ being Y or C₁ to C₆ alkyl, is reacted, in a first reaction step with an at least equimolar amount of a HO-C₄₋₈ alkylenal, preferably HO-C₆ alkylenal, and in an optional second reaction step with an at least equimolar amount of a HO-C₄₋₈ alkylenal, preferably a HO-C₆ alkylenal, which HO-C₄₋₈ alkylenal is the same or different HO-C₄₋₈ alkylenal as in the previous reaction step, to obtain a product as represented by the Formula (II), 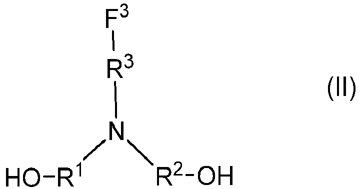 and wherein the obtained reaction product of Formula (II), wherein R₁, R₂, R₃ and F³ have the meanings as defined before, is reacted with an at least equimolar amount of a carboxylic acid of Formula (III) having a stereogenic center C* in the (R) form or in the (S) form: 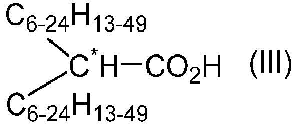
wherein the C₆₋₂₄ alkyl groups on the stereogenic center C* are different, and in an optional further reaction step with an at least equimolar amount of a carboxylic acid of Formula (III) having a stereogenic center C* in the (R) form or in the (S) form, wherein the C₆₋₂₄ alkyl groups on the stereogenic center C* are different, which carboxylic acid of Formula (III) is the same or different carboxylic acid of Formula (III) as in the previous reaction step.

Though the inventive process is exemplified for a limited number of functional groups, the skilled man can easily adapt the process to other functional groups and protective groups, if needed, on the basis of the understanding of the inventive process and his chemical skills.

In a more specific embodiment, the present invention is directed to a process for preparing a lipid compound as represented by Formula (I) wherein a X-C₂-C₄ alkylene-NH₂, wherein X is an -OY or -NR⁴₂, Y being a protective group and R⁴ being Y or C₁ to C₆ alkyl, is reacted in a first reaction step with an at least twofold equimolar amount of a HO-C₄₋₈ alkylenal to obtain a product as represented by the Formula (II), wherein R₃ is C₂ to C₄ alkylene and R¹ and R² are the same and are each C₄₋₈ alkylene, and the obtained reaction product of Formula (II) is reacted in a further reaction step with an at least twofold equimolar amount of a carboxylic acid of Formula (IIIa) having a stereogenic center C* in the (R) form or in the (S) form:

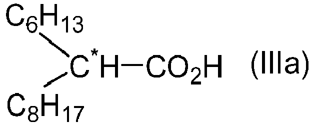

By using at least the twofold equimolar amount in the two reaction steps, a "symmetrical" lipid compound with two stereogenic centers only can be obtained.

As a key compound, the starting compound as represented by Formula (IV) can be used in the inventive process as for such compound, each lipid compound covered by the main claim can be obtained by reacting said starting compound with the appropriate reaction partner and subjecting the obtained reaction product to suitable further treatment and purifying steps: 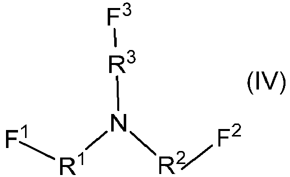 wherein R¹ and R² are the same or different and are each C₄₋₈ alkylene;
R₃ is C₁-C₂₄ alkylene;
F¹ and F² are each selected from -(C=O)OH, -C(=O)H, -OH, -S(O)_{1,2}H, -S-S-H, -C(=O)SH, -C(=S)OH, -RC(=O)H, -C(=O)R, -O-C(=O)R or a direct bond, wherein R being H or C₁ to C₁₂ alkyl, and
F³ is -OY or -NR⁴₂, Y being a protective group and R⁴ being Y or C₁ to C₆ alkyl.

Thus, the present invention is also directed to said compound of Formula (IV), and more particularly also to the more specified starting compound as represented by Formula (II) 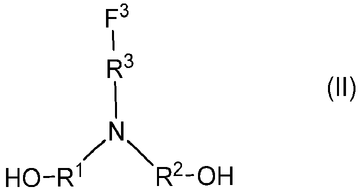 wherein R¹ and R² are the same or different and are each C₄₋₈ alkylene;
R₃ is C₁-C₂₄ alkylene; and
F³ is -OY or -NR⁴₂, Y being a protective group and R⁴ being Y or C₁ to C₆ alkyl.

In the inventive process, any reaction partner or reacting compound having one or more stereogenic center will be used in its enriched form, idealy enantiopure form, where the one or more stereogenic center(s) is present in one of the two forms (R)-form or (S)-form, The respective enriched form may be obtained by synthesis or resolution.

In the inventive process, the choice of the organic solvent is not critical as long as it is an aprotic organic solvent selected from THF, acetonitrile, other nitriles, chlorinated hydrocarbons, or other aprotic solvents, or mixtures thereof. The reaction conditions are also not critical and the reaction is usually carried out at a temperature between -78°C and 50°C, preferably -40°C to 30°C, under an non-reactive atmosphere under ambient pressure.

In the context of the aspects of the present invention, the following definitions are more general terms which are used throughout the present application.

When a range of values is listed, it is intended to encompass each value and sub-range within the range. For example "C₁₋₆" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

As used herein, "alkyl" refers to a radical of a straight-chain, branched or cyclic saturated hydrocarbon group having from 1 to 24 carbon atoms ("C₁₋₂₄ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 4 to 8 carbon atoms ("C₄₋₈ alkyl"), in others 1 to 6 carbon atoms. Examples of C₁₋₆ alkyl groups include methyl (C₁), ethyl (C₂), n-propyl (C₃), isopropyl (C₃), n-butyl (C₄), tert-butyl (C₄), sec-butyl (C₄), isobutyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently unsubstituted (an "unsubstituted alkyl") or substituted (a "substituted alkyl") with one or more substituents. In certain embodiments, the alkyl group is an unsubstituted alkyl (*e.g*., -CH₃). In certain embodiments, the alkyl group is a substituted alkyl.

"Alkenyl" refers to a radical of a straight-chain or branched hydrocarbon group having from 2 to 24 carbon atoms, and one or more carbon-carbon double bonds("C₂₋₂₄ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like. Unless otherwise specified, each instance of an alkenyl group is independently optionally substituted, *i.e.,* unsubstituted (an "unsubstituted alkenyl") or substituted (a "substituted alkenyl") with one or more substituents. In certain embodiments, the alkenyl group is unsubstituted C₂₋₁₀ alkenyl. In certain embodiments, the alkenyl group is substituted C₂₋₁₀ alkenyl.

The present invention is further illustrated by the following Figures. In the Figures, it is shown:
- Figure 1: Conventional Synthesis of ALC-315
- Figure 2A: Inventive Synthesis of (S,S)-ALC-0315

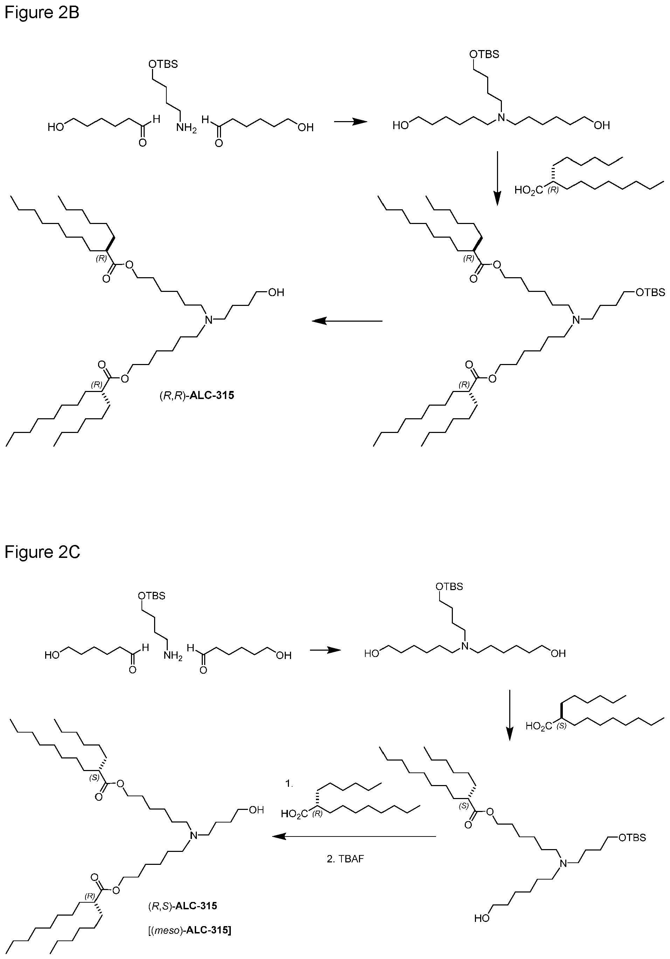

- Figure 2B: Inventive Synthesis of (R,R)-ALC-0315
- Figure 2C: Inventive Synthesis of (S,R)-ALC-0315 ((meso)-ALC-0315)

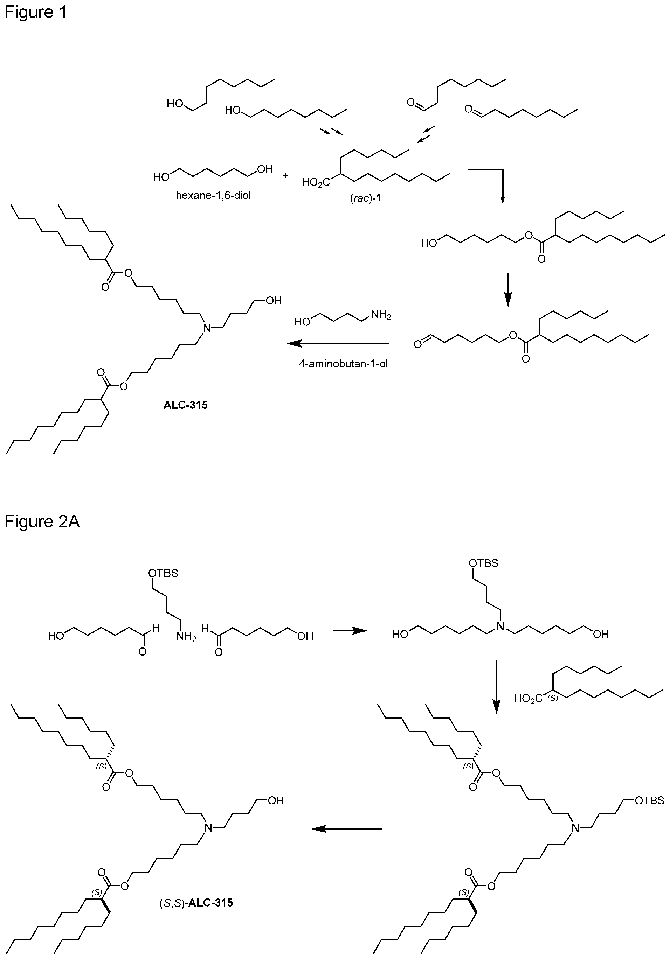

The synthesis of ALC-315, as used in the prior art and illustrated in Figure 1, starts from commercially available alpha-branched acid (rac)-1. Such acids which, in the alpha-position are substituted with two n-alkyl chains that differ by an element of ethylene (CH₂CH₂), are technically produced in a dimerization of two aldehydes of alcohols and are abundant and inexpensive. However, in their technical synthesis, the produced acids are racemic, which means a mixture of the two mirror image forms described as (R)- and (S)-enantiomers.

In the first step of the synthesis of ALC-315, (*rac*)-**1** is esterified with 1,6-hexanediol. The resulting alcohol is oxidized to the corresponding aldehyde, which in turn undergoes a double reductive amination with 4-aminobutan-1-ol to furnish ALC-315. Said process is less favorable in view of its restriction to obtain various enantiopure compounds.

The approach of the inventors starts from an enantiopure carboxylic acid, either from its (*R*)- or from (*S*)-form. The enantiopure acid in turn could be obtained either via separation of the racemate by physical or chemical methods, or obtained in an enantioselective synthesis. With the enantiopure acid **1**, either (*R,R*)-ALC-0315 and (*S,S*)-ALC-0315 can then be accessed following the known synthesis of the ALC-315 isomers, or an alternative synthesis. The synthesis of the corresponding (*meso*)-ALC-315 would require a modified synthesis, in which the two enantiomers of acid **1**, will have to be introduced in two separate steps.

In the present application, the inventors describe the synthesis of all possible stereoisomers of LNPs such as ALC-315 using the approach on the basis of their considerations, in which the chiral acid **1** is first prepared in an enantiomerically pure form. Following this, the three ALC-315 isomers are prepared separately in a modified procedure. In one embodiment, the chiral acid is obtained via a chiral auxiliary approach, in which a chiral amide can be alkylated in the alpha position diastereoselectively. The amide is the hydrolyzed to furnish the desired acid **1.** Accordingly, (+)-pseudoephedrine is first converted to the corresponding amide with caprylic acid chloride. The amide is the alkylated with 1-iodooctane, followed by hydrolysis to obtain enantioenriched (*R*)-**1** with an enantiomeric ratio (e.r.) of 98:2. The enantiomer of (*R*)-**1**, (*S*)-**1** can be accessed similarly: By first reacting (+)-pseudoephedrine with capric anhydride, alkylating the resulting amide with 1-iodohexane, followed by hydrolysis. In this case, (*S*)-**1** is obtained with an e.r. of 98.5:1.5.

In another embodiment, racemic acid **1** is separated by high-performance liquid chromatography. Accordingly, the inventors found that *rac*-**1** can be separated on a preparative scale using HPLC with a chiral stationary phase (Chiralpak IG-3).

In a further embodiment, racemic acid **1** is converted to the corresponding amide and separated by high-performance liquid chromatography. Accordingly, the inventors found that *rac*-**1** in its amide form can be separated on a preparative scale using HPLC with a chiral stationary phase (Chiralpak IG-3).

With enantiopure acids (*S*)-**1** and (*R*)-**1** in hand, the inventors synthesized the corresponding ALC-315 isomers following a newly developed route. Accordingly, 6-hydroxyhexanal is reductively aminated with 4-((tert-butyldimethylsilyl)oxy)butan-1-amine. The resulting diol is then doubly esterified with acid (*S*)-1. Removal of the silyl group then provides the pure product (*S,S*)-ALC-0315. Its enantiomer, (*R,R*)-ALC-0315, has been prepared following the same procedure but using acid (*R*)-**1** instead of acid (*S*)-1.

Finally, (*R,S*)-ALC-0315 (or (*meso*)-ALC-0315) has also been prepared. Accordingly, the silyl-protected diol described above is subsequently esterified, in a first step with (*S*)-**1**, and in a second step with (*R*)-**1**. Finally, the silyl group is removed to obtain the pure product (*meso*)-ALC-0315.

The present invention is further illustrated by the following Experimental Part.

### Experimental Part

### Example 1: Synthesis of (R)-2-hexyldecanoic acid

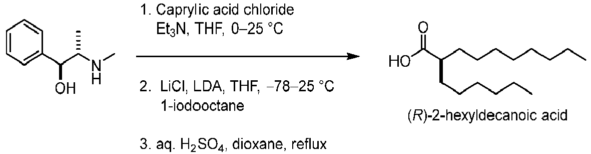

**Step 1:** A flame dried 250 mL two-neck flask was charged with (+)-pseudoephedrine (4.0 g, 24.2 mmol, 1.0 equiv.) and triethylamine (4.8 mL, 27.8 mmol, 1.3 equiv.) followed by addition of anhydrous tetrahydrofuran (50 mL). The reaction mixture was stirred and cooled to 0 °C. An ice-cold solution of caprylic acid chloride (4.8 mL, 27.8 mmol, 1.15 equiv.) in tetrahydrofuran (10 mL) was added via addition funnel over 10 min. After 20 min, excess acid chloride was quenched by the addition of water (10 mL). The reaction mixture was then partitioned between ethyl acetate (100 mL) and brine (80 mL), and the organic layer was separated. The organic layer was extracted with brine (2 x 80 mL), dried over Na₂SO₄ and concentrated. Upon evaporation of the solvent under reduced pressure, N-((*1S*,*2S*)-1-hydroxy-1-phenylpropan-2-yl)-N-methyloctanamide was obtained as an oil (6.0 g, 85% yield).

**Step 2:** A flame dried 250 mL two-neck flask was charged with a magnetic stirrer, lithium chloride (3.2 g, 76.1 mmol, 6.0 equiv.), diisopropylamine (4.0 mL, 28.6 mmol, 2.25 equiv.), and anhydrous tetrahydrofuran (15 mL). The suspension was cooled to -78 °C. After 10 min, a solution of n-butyllithium in hexanes (2.8 M, 9.4 mL, 26.4 mmol, 2.08 equiv.) was added. The reaction mixture was warmed to 0 °C for 10 min and then cooled to -78 °C. An ice-cold solution of N-((*1S*,*2S*)-1-hydroxy-1-phenylpropan-2-yl)-N-methyloctanamide (3.7 g, 12.7 mmol, 1.0 equiv.) in tetrahydrofuran (55 mL) was added slowly. The reaction mixture was stirred at-78 °C for 1 h, at 0 °C for 15 min, and at room temperature for 5 min. At 0 °C, 1-iodooctane (17.7 mL, 149 mmol, 1.50 equiv.) was added and stirring was continued for additional 15 min at 0 °C. Then it was quenched by the addition of saturated aq. NH₄Cl solution. The mixture was partitioned between saturated aq. NH₄Cl solution (80 mL) and ethyl acetate (100 mL). The aqueous layer was separated and extracted with ethyl acetate (3 x 100 mL). The combined organic layers were dried over Na₂SO₄ and concentrated. The crude product was purified by flash column chromatography on silica (eluent: 5% EtOAc to 15% EtOAc in hexanes *v*/*v*). Upon evaporation of the solvent under reduced pressure, (*R*)-2-hexyl-N-((*1S*,*2S*)-1-hydroxy-1-phenylpropan-2-yl)-N-methyldecanamide was obtained as an oil (2.9 g 56% yield).

**Step 3:** A 50 mL one-neck flask was charged with a magnetic stirrer, (*R*)-2-hexyl-N-((*1S*,*2S*)-1-hydroxy-1-phenylpropan-2-yl)-N-methyldecanamide (1.2 g, 3.0 mmol, 1.0 equiv.), and 1,4-dioxane (6 mL). Aq. 18 M H₂SO₄ acid solution (6 mL) was added slowly. The reaction mixture was heated at 110 °C for 1 h. After 1 h the reaction mixture was cooled to 0 °C and basified to pH >10 using 50% (w/w) aqueous sodium hydroxide solution. The resulting mixture was partitioned between water (10 mL) and dichloromethane (20 mL). The aqueous layer was separated and extracted with dichloromethane (2 x 10 mL). The aqueous layer was acidified to pH < 2 using 6 M aq. H₂SO₄ acid solution, extracted with dichloromethane (3 x 20 mL) and the latter organic extracts were dried over Na₂SO₄. It was then concentrated and the crude product was purified by flash column chromatography on silica (eluent: 20% ethyl acetate in hexanes *v*/*v*). Upon evaporation of the solvent under reduced pressure, (R)-2-hexyldecanoic acid was obtained as an oil (0.23 g, 35% yield). The enantiomeric ratio was measured by HPLC, Chiralpak IG-3, acetonitrile/0.1% TFA-water = 65:35 (v/v), flow rate = 1.0 mL/min, λ = 220 nm, 298 K, t_{R} = 14.7 min (major) and t_{R} = 16.1 min (minor). er = 98:2.

### Example 2: Synthesis of (S)-2-hexyldecanoic acid

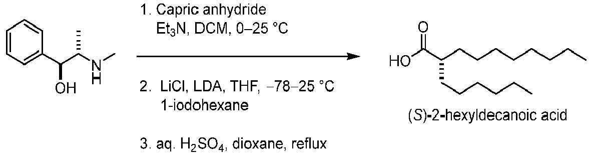

**Step 1:** A flame dried 100 mL two-neck flask was charged with (+)-pseudoephedrine (2.0 g, 12.1 mmol, 1.0 equiv.) and triethylamine (2.1 mL, 14.5 mmol, 1.2 equiv.) followed by addition of anhydrous dichloromethane (24 mL). At rt, capric anhydride (4.8 mL, 13.0 mmol, 1.1 equiv.) was added over 10 min. After 3 h, excess anhydride was quenched by the addition of water (1 mL). The reaction mixture was then partitioned between ethyl acetate (50 mL) and brine (40 mL), and the organic layer was separated. The organic layer was extracted with brine (2 x 20 mL), dried over Na₂SO₄ and concentrated. Upon evaporation of the solvent under reduced pressure, N-((*1S*,*2S*)-1-hydroxy-1-phenylpropan-2-yl)-N-methyldecanamide was obtained as an oil (3.5 g, 95% yield).

**Step 2:** A flame dried 250 mL two-neck flask was charged with a magnetic stirrer, lithium chloride (2.1 g, 69.5 mmol, 6.0 equiv.), diisopropylamine (3.7 mL, 26 mmol, 2.25 equiv.), and anhydrous tetrahydrofuran (14 mL). The suspension was cooled to -78 °C. After 10 min, a solution of n-butyllithium in hexanes (2.8 M, 8.6 mL, 24 mmol, 2.08 equiv.) was added. The reaction mixture was warmed to 0 °C for 10 min and then cooled to -78 °C. An ice-cold solution of N-((*1S*,*2S*)-1-hydroxy-1-phenylpropan-2-yl)-N-methyldecanamide (3.7 g, 11.6 mmol, 1.0 equiv.) in THF (50 mL) was added slowly. The reaction mixture was stirred at -78 °C for 1 h, at 0 °C for 15 min, and at room temperature for 5 min. At 0 °C, 1-iodohexane (2.6 mL, 17.4 mmol, 1.50 equiv.) was added and stirring was continued for additional 15 min at 0 °C. Then it was quenched by the addition of saturated aq. NH₄Cl solution. The mixture was partitioned between saturated aq. NH₄Cl solution (80 mL) and ethyl acetate (50 mL). The aqueous layer was separated and extracted with ethyl acetate (3 x 30 mL). The combined organic layers were dried over Na₂SO₄ and concentrated. The crude product was purified by flash column chromatography on silica (eluent: 5% EtOAc to 15% EtOAc in hexanes *v*/*v*). Upon evaporation of the solvent under reduced pressure, (S)-2-hexyl-N-((*1S*,*2S*)-1-hydroxy-1-phenylpropan-2-yl)-N-methyldecanamide was obtained as an oil (2.2 g, 47% yield).

**Step 3:** A 50 mL one-neck flask was charged with a magnetic stirrer, (S)-2-hexyl-N-((*1S*,*2S*)-1-hydroxy-1-phenylpropan-2-yl)-N-methyldecanamide (0.94 g, 2.3 mmol, 1.0 equiv.) and 1,4-dioxane (5 mL). Aq. 18 M H₂SO₄ acid solution (5 mL) was added slowly. The reaction mixture was heated at 110 °C for 1 h. After 1 h the reaction mixture was cooled to 0 °C and basified to pH >10 using 50% (w/w) aqueous sodium hydroxide solution. The resulting mixture was partitioned between water (10 mL) and dichloromethane (20 mL). The aqueous layer was separated and extracted with dichloromethane (2 x 10 mL). The aqueous layer was acidified to pH < 2 using 6 M aq. H₂SO₄ acid solution, extracted with dichloromethane (3 x 20 mL) and the latter organic extracts were dried over Na₂SO₄. It was then concentrated and the crude product was purified by flash column chromatography on silica (eluent: 20% ethyl acetate in hexanes *v*/*v*). Upon evaporation of the solvent under reduced pressure, (S)-2-hexyldecanoic acid was obtained as an oil (0.25 g, 42% yield). The enantiomeric ratio was measured by HPLC, Chiralpak IG-3, acetonitrile/0.1 % TFA-water = 65:35 (v/v), flow rate = 1.0 mL/min, λ = 220 nm, 298 K, t_{R} = 15.0 min (minor) and t_{R} = 15.4 min (major). er = 98.5:1.5.

### Example 3: Preparative HPLC separation of rac-2-hexyldecanoic acid

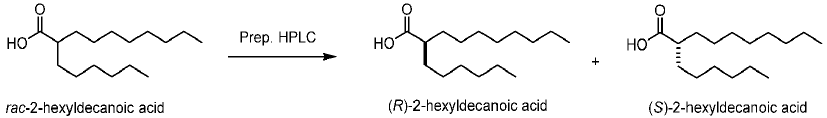

*rac-*2-hexyldecanoic acid was purchased from Sigma-Aldrich and was used as arrived. *rac-*2-hexyldecanoic acid was successfully separated on a preparative scale using HPLC. **Condition:** Chiralpak IG-3, acetonitrile/0.1 % TFA-water = 65:35 (v/v), flow rate = 1.0 mL/min, A = 220 nm, 298 K, t_{R} = 16.0 min and t_{R} = 16.8 min.

### Example 4: Preparative HPLC separation of amide derivatives:

*rac*-2-hexyldecanoic acid was converted into the corresponding acid chloride (CICOCOCI, cat. DMF, DCM, rt, 2h), which was reacted with several different chiral and achiral amines (see below graphic). Then the corresponding amides were separated using HPLC (chiral and achiral stationary phases). After hydrolysis (as described in step 3), the enantiopure 2-hexyldecanoic acids can be obtained.

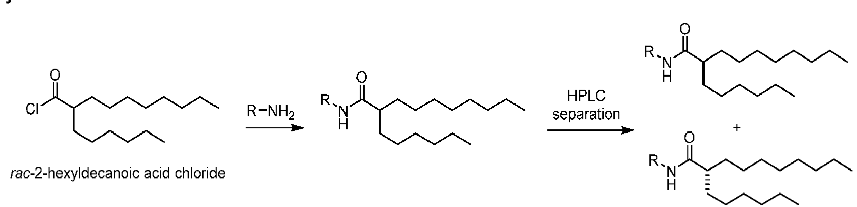

### Example 5: Synthesis of 6,6'-((4-((tert-butyldimethylsilyl)oxy) butyl)azanediyl)bis(hexan-1-ol)

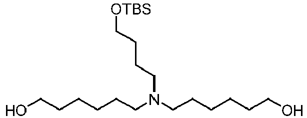

A 250 mL one-neck flask was charged with magnetic stirrer, 4-((tertbutyldimethylsilyl)oxy)butan-1-amine hydrochloride (4.8 g, 20.0 mmol, 1.0 equiv.) and 6-hydroxyhexanal (5.8 g, 50 mmol, 2.5 equiv.). Acetic acid (4.8 g, 80 mmol, 4.0 eq.) and 1,2-dichloroethane (63.0 mL) were added at rt. The reaction mixture was stirred at room temperature for 10 min. Then to the reaction mixture NaHB(OAc)₃ (17.0 g, 80 mmol, 4.0 equiv.) was added in small portions over 1 h at rt. After complete addition of NaHB(OAc)₃, the reaction mixture was further stirred at room temperature for overnight. The reaction mixture was neutralized with saturated aq. NaHCO₃ solution and extracted with ethyl acetate (3 x 100 mL). The combined organic layers were washed with brine dried over Na₂SO₄ and concentrated. The crude product was purified by flash column chromatography on silica (eluent: 100% acetone). Upon evaporation of the solvent under reduced pressure, 6,6'-((4-((tert-butyldimethylsilyl)oxy)butyl)azanediyl)bis(hexan-1-ol) was obtained as a light yellow oil (4.1 g, 50% yield).

### Example 6: Synthesis of 6-((4-((tert-butyldimethylsilyl)oxy)butyl)(4-hydroxybutyl)amino)hexan-1-ol

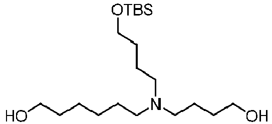

A 25 mL one-neck flask was charged with magnetic stirrer, 4-((tertbutyldimethylsilyl)oxy)butan-1-amine hydrochloride (1.1 equiv.) and 6-hydroxyhexanal (1.0 equiv.). Acetic acid (2.0 eq.) and 1,2-dichloroethane (6.3 mL) were added at rt. The reaction mixture was stirred at room temperature for 10 min. Then to the reaction mixture NaHB(OAc)₃ (1.5 equiv.) was added in small portions over 10 min at rt. After complete addition of NaHB(OAc)₃, the reaction mixture was further stirred at room temperature for overnight. Then 4-hydroxybutanal (1.1 equiv.), acetic acid (2.0 eq.) were added at rt. The reaction mixture was stirred at room temperature for 10 min. Then to the reaction mixture NaHB(OAc)₃ (1.5 equiv.) was added in small portions over 10 min at rt. After complete addition of NaHB(OAc)₃, the reaction mixture was further stirred at room temperature for 6 h. The reaction mixture was neutralized with saturated aq. NaHCO₃ solution and extracted with ethyl acetate (3 x 10 mL). The combined organic layers were washed with brine dried over Na₂SO₄ and concentrated. The crude product was purified by flash column chromatography on silica (eluent: 100% acetone). Upon evaporation of the solvent under reduced pressure, 6-((4-((tert-butyldimethylsilyl)oxy)butyl)(4-hydroxybutyl)amino)hexan-1-ol was obtained as a light yellow oil.

### Example 7: Synthesis of ((4-hydroxybutyl)azanediyl)bis(hexane-6,1-diyl) (2R,2'R)-bis(2-hexyldecanoate)

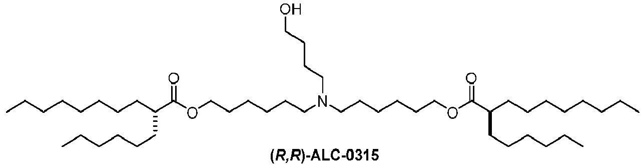

A flame dried 25 mL schlenk tube was charged with 6,6'-((4-((tertbutyldimethylsilyl)oxy)butyl)azanediyl)bis(hexan-1-ol) (39.4 mg, 0.1 mmol, 1.0 equiv.), (R)-2-hexyldecanoic acid (50.0 mg, 0.2 mmol, 2.0 equiv., 98:2 er) and DMAP (25.6 mg, 0.24 mmol, 2.4 equiv.) followed by addition of anhydrous dichloromethane (3 mL). The reaction mixture was cooled to 0 °C and 1-ethyl-3-(3-dimethyllaminopropyl)carbodiimide hydrochloride (44.9 mg, 0.24 mmol, 2.4 equiv.) was added. The mixture was allowed to stir overnight at rt. The reaction mixture was then diluted with saturated aq. sodium bicarbonate solution (2 mL), brine (5 mL) and extracted with dichloromethane (3 x 5 mL). The combined organics were dried over Na₂SO₄ and concentrated under reduced pressure. The crude product was purified by flash column chromatography on silica (eluent: 40% ethyl acetate in hexanes *v*/*v*). Upon evaporation of the solvent under reduced pressure, ((4-((tertbutyldimethylsilyl)oxy)butyl)azanediyl)bis(hexane-6,1-diyl) (2*R*,2'*R*)-bis(2-hexyldecanoate) was obtained as an oil (75.0 mg, 87% yield).

((4-((tert-butyldimethylsilyl)oxy)butyl)azanediyl)bis(hexane-6,1-diyl) (2*R*,2'*R*)-bis(2-hexyldecanoate) was dissolved in tetrahydrofuran (5 mL) and 1 M tetrahydrofuran solution of TBAF (0.4 mL, 0.4 mmol, 4.0 equiv.) was added at rt. After 2 h, the reaction mixture was quenched with water and extracted with dichloromethane (3 x 5 mL). The combined organic layers were dried over Na₂SO₄ and concentrated under reduced pressure. The crude product was purified by flash column chromatography on silica (eluent: 0% to 30% acetone in ethyl acetae *v*/*v*). Upon evaporation of the solvent under reduced pressure, ((4-hydroxybutyl)azanediyl)bis(hexane-6,1-diyl) (2*R*,2'*R*)-bis(2-hexyldecanoate) was obtained as an oil (51.0 mg, 68% yield over two steps).

**Determination of enantiomeric ratio:** The enantiomeric ratio was measured by HPLC after derivatizations of the lipid to carboxylic acid following the reaction sequence as below: 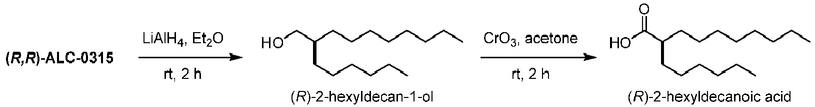 er ≥ 95.5:4.5 and calculated dr = >20:1

### Example 8: Synthesis of ((4-hydroxybutyl)azanediyl)bis(hexane-6,1-diyl) (2S,2'S)-bis(2-hexyldecanoate)

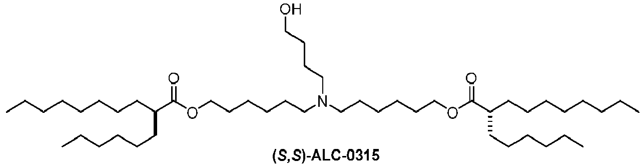

Following the procedure for example **4** and using (S)-2-hexyldecanoic acid, ((4-hydroxybutyl)azanediyl)bis(hexane-6,1-diyl) (2S,2'S)-bis(2-hexyldecanoate) was obtained as an oil (61.0 mg, 77% yield over two steps). er ≥ 98:2 and calculated dr = >20:1

### Example 9: Synthesis of 6-((6-(((R)-2-hexyldecanoyl)oxy)hexyl)(4-hydroxybutyl)amino)hexyl (S)-2-hexyldecanoate

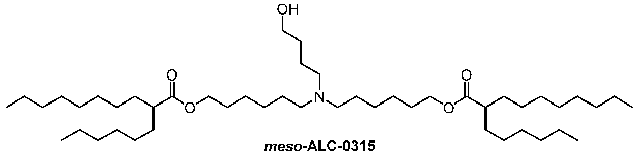

A flame dried 25 mL schlenk tube was charged with 6,6'-((4-((tertbutyldimethylsilyl)oxy)butyl)azanediyl)bis(hexan-1-ol) (75.6 mg, 0.19 mmol, 2.0 equiv.), (S)-2-hexyldecanoic acid (25.0 mg, 0.1 mmol, 1.0 equiv., 98:2 er) and DMAP (13.7 mg, 0.11 mmol, 1.2 equiv.) followed by addition of anhydrous dichloromethane (3 mL). The reaction mixture was cooled to 0 °C and 1-ethyl-3-(3-dimethyllaminopropyl)carbodiimide hydrochloride (21.5 mg, 0.11 mmol, 1.2 equiv.) was added. The mixture was allowed to stir overnight at rt. The reaction mixture was then diluted with saturated aq. sodium bicarbonate solution (2 mL), brine (5 mL) and extracted with dichloromethane (3 x 5 mL). The combined organics were dried over Na₂SO₄ and concentrated under reduced pressure. The crude product was purified by flash column chromatography on silica (eluent: 50% acetone in diethyl ether *v*/*v*). Upon evaporation of the solvent under reduced pressure, 6-((4-((tertbutyldimethylsilyl)oxy)butyl)(6-hydroxyhexyl)amino)hexyl (S)-2-hexyldecanoate was obtained as an oil.

The isolated 6-((4-((tert-butyldimethylsilyl)oxy)butyl)(6-hydroxyhexyl)amino)hexyl (S)-2-hexyldecanoate was then transferred to a flame dried 25 mL schlenk tube. (R)-2-hexyldecanoic acid (25.0 mg, 0.1 mmol, 1.0 equiv., 98:2 er) and DMAP (13.7 mg, 0.11 mmol, 1.2 equiv.) were added followed by addition of anhydrous dichloromethane (3 mL). The reaction mixture was cooled to 0 °C and 1-ethyl-3-(3-dimethyllaminopropyl)carbodiimide hydrochloride (21.5 mg, 0.11 mmol, 1.2 equiv.) was added. The mixture was allowed to stir overnight at rt. The reaction mixture was then diluted with saturated aq. sodium bicarbonate solution (2 mL), brine (5 mL) and extracted with dichloromethane (3 x 5 mL). The combined organics were dried over Na₂SO₄ and concentrated under reduced pressure. The crude product was purified by flash column chromatography on silica (eluent: 40% ethyl acetate in hexanes *v*/*v*). Upon evaporation of the solvent under reduced pressure, 6-((4-((tert-butyldimethylsilyl)oxy)butyl)(6-(((R)-2-hexyldecanoyl)oxy)hexyl)amino)hexyl (S)-2-hexyldecanoate was obtained as an oil (66 mg, 80% yield over two steps).

6-((4-((tert-butyldimethylsilyl)oxy)butyl)(6-(((R)-2-hexyldecanoyl)oxy)hexyl)amino) hexyl-(S)-2-hexyldecanoate was dissolved in tetrahydrofuran (5 mL) and 1 M tetrahydrofuran solution of TBAF (0.4 mL, 0.4 mmol, 4.0 equiv.) was added at rt. After 2 h, the reaction mixture was quenched with water and extracted with dichloromethane (3 x 5 mL). The combined organic layers were dried over Na₂SO₄ and concentrated under reduced pressure. The crude product was purified by flash column chromatography on silica (eluent: 0% to 30% acetone in EtOAc *v*/*v*). Upon evaporation of the solvent under reduced pressure, 6-((6-(((R)-2-hexyldecanoyl)oxy)hexyl)(4-hydroxybutyl)amino)hexyl (S)-2-hexyldecanoate was obtained as an oil (51.0 mg, 71% yield over three steps). calculated dr = >20:1

## Claims

1. A lipid compound as represented by the following Formula (I): 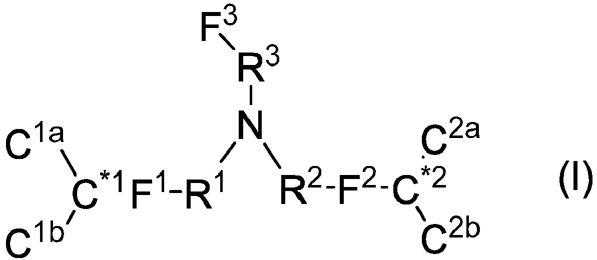 or a pharmaceutically acceptable salt thereof, wherein:
C*¹ and C^{*2} are each representing a CH group;
C^{1a} and C^{1b} are different from each other and independently represent C₆-C₂₄ alkyl or C6-C24 alkenyl;
C^{2a} and C^{2b} are different from each other and independently represent C₆-C₂₄ alkyl or C6-C24 alkenyl;
C^{1a} is the same or different as C^{2a} and C^{1b} is the same or different as C^{2b};
R¹ and R² are each unsubstituted C₁-C₁₂ alkylene or C₂- C₁₂ alkenylene, R¹ and R² preferably representing the same group;
R₃ is C₁-C₂₄ alkylene, C₂-C₂₄ alkenylene, C₃-C₈ cycloalkylene, or C₃-C₈ cycloalkenylene;
F¹ and F² are each selected from -O(C=O)-, -(C=O)O-, -C(=O)-, -O-, -S(O)_{1,2}-, -S-S-, -C(=O)S-, SC(=O)-, - RC(=O)-, -C(=O)R-, RC(=O)R-, -OC(=O)R-, - RC(=O)O- or a direct bond, wherein R being H or C₁ to C₁₂ alkyl, F¹ and F² preferably representing the same group and more preferably being -O(C=O)- or -(C=O)O-, respectively;
F³ is H, OR⁴, -NR⁴₂, -CN, halogen, -C(=O)O-(C₁-C₁₂ alkyl)-, (C₁-C₁₂ alkyl)-OC(=O)-(C₁-C₁₂ alkyl)- or - R⁴C(=O)-(C₁-C₁₂ alkyl)-, R⁴ being H or C₁ to C₆ alkyl, wherein the stereogenic centers at C*¹ and C*² are independently from each other enriched in one stereogenic form selected from the (R)-form or the (S)-form.

2. The lipid compound as claimed in claim 1, wherein, in Formula (I), C^{1a} and C^{2a} represent the same group selected from C₆-C₂₄ alkyl or C₆-C₂₄ alkenyl, and C^{1b} and C^{2b} represent the same group selected from C₆-C₂₄ alkyl or C₆-C₂₄ alkenyl.

3. The lipid compound as claimed in claim 1 or 2, wherein, in Formula (I), F1 and F2 represent the same group selected from -O(C=O)-, -(C=O)O-, -C(=O)-, -O-, -S(O)_{1,2}-, -S-S-, -C(=O)S-, SC(=O)-, - RC(=O)-, -C(=O)R-, RC(=O)R-, -OC(=O)R- or-RC(=O)O-, or a direct bond, wherein R being H or C₁ to C₁₂ alkyl,
preferably -O(C=O)- or -(C=O)O-, respectively.

4. The lipid compound as claimed in any one of the preceding claims, wherein, in Formula (I), R¹ and R² are the same and each unsubstituted C₁-C₁₂ alkylene.

5. The lipid compound as claimed in any one of the preceding claims, wherein, in Formula (I), R³ is C₁-C₂₄ alkylene.

6. The lipid compound as claimed in any one of the preceding claims, wherein, in Formula (I), F³ is OR⁴, -NR⁴₂, -CN, -or halogen, R⁴ being H or C₁ to C₆ alkyl.

7. The lipid compound as claimed in claim 1, wherein, in Formula (I),
C*¹ and C^{*2} are each representing a CH group;
C^{1a} and C^{2a} represent the same C₆-C₂₄ alkyl group;
C^{1b} and C^{2b} represent the same C₆-C₂₄ alkyl group;
F¹ and F² each represent the same group selected from -O(C=O)-, -(C=O)O-;
R¹ and R² are each C₄₋₈ alkylene;
R³ is C₁-C₂₄ alkylene; and
F³ is OR⁴, or -NR⁴₂, R⁴ being H or C₁ to C₆ alkyl,
wherein the stereogenic centers at C*¹ and C*² are independently from each other enriched in one stereogenic form selected from the (R)-form or the (S)-form.

8. The lipid compound as claimed in claim 1, wherein, in Formula (I),
C*¹ and C^{*2} are each representing a CH group;
C^{1a} and C^{2a} each represent a C₆ alkyl group;
C^{1b} and C^{2b} each represent a C₈ alkyl group;
F1 and F2 each represent -O(C=O)- group bound to C*^{(1,2)};
R¹ and R² are each unsubstituted C₄₋₈ alkylene, preferably C₆ alkylene; R³ is C₂ to C₄ alkylene; and
F³ is OR⁴, or -NR⁴₂, R⁴ being H or a methyl group,
wherein the stereogenic centers at C*¹ and C*² are independently from each other enriched in one stereogenic form selected from the (R)-form or the (S)-form.

9. Process for preparing a lipid compound as represented by the following Formula (I): 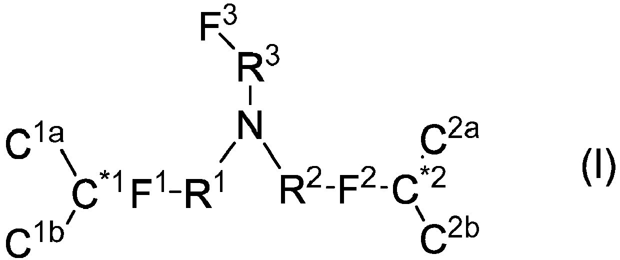 wherein, in Formula (I),
C*¹ and C^{*2} are each representing a CH group;
C^{1a} and C^{2a} represent the same group selected from C₆-C₂₄ alkyl, and C^{1b} and C^{2b} represent the same group selected from C₆-C₂₄ alkyl;
F¹ and F² each represent the same group selected from -O(C=O)-, -(C=O)O-;
R¹ and R² are each C₄₋₈ alkylene;
R³ is C₁-C₂₄ alkylene; and
F³ is OR⁴, or -NR⁴₂, R⁴ being H or C₁ to C₆ alkyl,
wherein the stereogenic centers at C*¹ and C*² are independently from each other enriched in one stereogenic form selected from the (R)-form or the (S)-form, wherein a X-C₁-C₂₄ alkylene-NH₂, wherein X is an -OY or -NR⁴₂, Y being a protective group and R⁴ being Y or C₁ to C₆ alkyl, is reacted, in a first reaction step with an at least equimolar amount of a HO-C₄₋₈ alkylenal, preferably HO-C₆ alkylenal, and in an optional second reaction step with an at least equimolar amount of a HO-C₄₋₈ alkylenal, preferably a HO-C₆ alkylenal, which HO-C₄₋₈ alkylenal is the same or different HO-C₄₋₈ alkylenal as in the previous reaction step, to obtain a product as represented by the Formula (II), 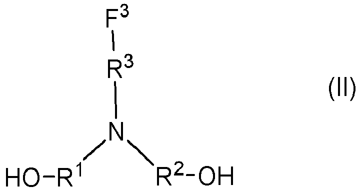
the obtained reaction product of Formula (II), wherein R₁, R₂, R₃ and F³ have the meanings as defined before in this claim, is reacted with an at least equimolar amount of a carboxylic acid of Formula (III) having a stereogenic center C* in the (R) form or in the (S) form: 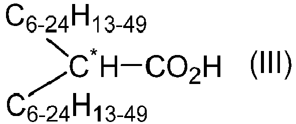
wherein the C₆₋₂₄ alkyl groups on the stereogenic center C* are different, and in an optional further reaction step with an at least equimolar amount of a carboxylic acid of Formula (III) having a stereogenic center C* in the (R) form or in the (S) form, wherein the C₆₋₂₄ alkyl groups on the stereogenic center C* are different, which carboxylic acid of Formula (III) is the same or different carboxylic acid of Formula (III) as in the previous reaction step.

10. Process for preparing a lipid compound as represented by Formula (I) according to claim 9 wherein a X-C₂-C₄ alkylene-NH₂, wherein X is an -OY or -NR⁴₂, Y being a protective group and R⁴ being Y or C₁ to C₆ alkyl, is reacted in a first reaction step with an at least twofold equimolar amount of a HO-C₄₋₈ alkylenal to obtain a product as represented by the Formula (II), wherein R³ is C₂ to C₄ alkylene and R¹ and R² are the same and are each C₄₋₈ alkylene, and the obtained reaction product of Formula (II) is reacted in a further reaction step with an at least twofold equimolar amount of a carboxylic acid of Formula (IIIa) having a stereogenic center C* in the (R) form or in the (S) form:

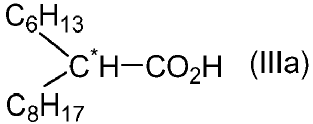

11. A starting compound as represented by Formula (IV) 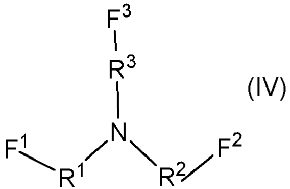
Wherein R¹ and R² are the same or different and are each C₄₋₈ alkylene;
R³ is C₁-C₂₄ alkylene;
F¹ and F² are each selected from -(C=O)OH, -C(=O)H, -OH, -S(O)_{1,2}H, -S-S-H,
-C(=O)SH, -C(=S)OH, -RC(=O)H, -C(=O)R, -O-C(=O)R or a direct bond, wherein R being H or C₁ to C₁₂ alkyl, and
F³ is -OY or -NR⁴₂, Y being a protective group and R⁴ being Y or C₁ to C₆ alkyl.

12. A starting compound as represented by Formula (II) 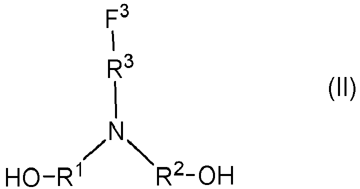
wherein R¹ and R² are the same or different and are each C₄₋₈ alkylene;
R³ is C₁-C₂₄ alkylene; and
F³ is -OY or -NR⁴₂, Y being a protective group and R⁴ being Y or C₁ to C₆ alkyl.
